# FASCICULE DE BREVET EUROPEEN

(11) **EP 4 248 919 B1**
(45) Date de publication et mention de la délivrance du brevet: **04.09.2024**
(21) Numéro de dépôt: 22305329.9
(22) Date de dépôt: 21.03.2022
(51) Int. Cl.: A61F 5/058, A61F 5/56

(54) **DISPOSITIF POUR LE TRAITEMENT ET LA PRÉVENTION DU BRUXISME, PROCÉDE DE MISE EN OEUVRE D'UN TEL DISPOSITIF**
VORRICHTUNG FÜR DIE BEHANDLUNG UND PRÄVENTION VON BRUXISMUS, VERFAHREN ZUR ANWENDUNG EINER SOLCHEN VORRICHTUNG
DEVICE FOR TREATMENT AND PREVENTION OF BRUXISM, METHOD FOR IMPLEMENTING SUCH A DEVICE

(43) Date de publication de la demande: 27.09.2023
(73) Titulaire: Bruxless, 13100 Aix-en-Provence (FR)
(72) Inventeur: BENSUSSAN, Romain, 13100 SAINT-MARC-JAUMEGARDE (FR); COLLINOT, Guillaume, 13820 ENSUES-LA-REDONNE (FR); CATELAS, Fabrice, 84120 PERTUIS (FR)
(74) Mandataire: IPAZ

(56) Documents cités:
- US-A- 6 117 092
- US-A1- 2019 313 934

## Description

L'invention concerne un dispositif et un procédé permettant de lutter contre le bruxisme.

Le bruxisme est un trouble dentaire qui correspond à un mouvement inconscient, diurne ou nocturne, des maxillaires en dehors de la phase de mastication.

Cela se traduit par un serrement des dents les unes contre les autres, qui peut être bruyant ou silencieux, et qui conduit à des conséquences plus ou moins importantes suivant les individus, comme une usure des dents ou des troubles au niveau de l'articulation temporo-mandibulaire pouvant créer des tensions annexes handicapantes au niveau de la nuque.

Il est plus compliqué de lutter contre le bruxisme nocturne qui est complètement inconscient : il existe des dispositifs qui permettent de limiter les conséquences de ce trouble sur les dents. Par exemple, des gouttières dentaires, conformées à la dentition de la personne, peuvent être portées pendant la nuit pour protéger les dents et en limiter l'usure. Même si de telles gouttières permettent de limiter l'usure des dents, elles n'agissent pas sur les causes du bruxisme et ne permettent pas de le traiter ou de le prévenir. Par ailleurs, elles sont inconfortables et un apprentissage est nécessaire pour s'endormir avec.

D'autres dispositifs ont été imaginés et ne se placent pas dans la bouche des personnes : la demande de brevet FR2618327 propose un dispositif qui détecte les sons ou vibrations émises par la personne grinçant des dents et qui, en réponse à ces sons ou vibrations détectées, émet un stimulus réflexogène ou dérangeant permettant de stopper le trouble. Un tel dispositif, qui se place à proximité de la tête de la personne atteinte de bruxisme, est compliqué à mettre en oeuvre pour être réellement efficace car de nombreux réglages doivent être faits pour que le dispositif soit efficace.

Il existe aussi des dispositifs d'analyse d'épisodes de bruxisme, tels que ceux décrits dans la demande EP 2 200 509, qui associent un examen de type électromyogramme (EMG) pour identifier une activité électrique des nerfs ou des muscles du visage (muscles temporaux et masséter), propres au bruxisme, à une stimulation électrique des muscles à travers des électrodes placées sur la peau d'une personne au niveau de ces muscles. On connaît encore, du document WO 97/43954, un appareil permettant de lutter contre le bruxisme, qui comprend un bandeau à placer autour de la tête d'une personne, équipé d'électrodes, qui se placent sur les muscles temporaux, et qui sont capables de détecter un phénomène de bruxisme en détectant des signaux (examen de type EMG également). Le bandeau est associé à un appareil auditif également porté par la personne, qui émet un signal audible par la personne pour lui signaler le phénomène de bruxisme en cours.

On connait également du document US 2019/0313934 un autre appareil qui comporte un bandeau à positionner sur le front d'une personne et qui est équipé d'une unité informatique qui comprend des capteurs de détection des tensions des muscles au niveau d'un front. Le dispositif est associé à une application smartphone.

L'invention propose une alternative aux dispositifs décrits ci-dessus, qui permet de réduire le phénomène de bruxisme en utilisant un dispositif à porter de préférence la nuit.

L'invention concerne à cet effet un dispositif pour aider à lutter contre le bruxisme, tel que défini par la revendication 1. Il comporte une structure conçue pour être portée sur la tête d'une personne, ladite structure comportant un bandeau présentant un plan de symétrie de part et d'autre duquel il s'étend.

Notamment, le dispositif conforme à l'invention est remarquable en ce que ladite structure comporte deux bras symétriques par rapport audit plan de symétrie, les deux dits bras étant reliés audit bandeau par une première extrémité de bras, et présentant une seconde extrémité libre de bras, ladite seconde extrémité libre de bras de chacun des bras étant apte à se positionner contre un muscle masséter quand ladite personne porte ladite structure et comportant un circuit masséter, apte à détecter une contraction du muscle masséter sur lequel ladite extrémité libre de bras est positionnée, ledit circuit masséter comportant un dispositif de vibrations apte à adopter soit un état de fonctionnement suivant lequel il émet des vibrations, soit un état de veille suivant lequel il n'émet pas de vibration, ledit dispositif comportant un module d'analyse et de commande, communiquant avec lesdits circuits masséter, et avec lesdits dispositifs de vibration, ledit module d'analyse et de commande étant apte à collecter et analyser des données relatives auxdites contractions détectées, et apte à commander le passage du dispositif de vibrations dudit état de veille audit état de fonctionnement, et inversement.

Ainsi conçu, le dispositif identifie des activités musculaires caractéristiques du bruxisme et offre une réponse à ces contractions quand elles sont identifiées comme caractérisant le phénomène de bruxisme. Les vibrations générées sont détectées par le réseau neuronal des muscles masséters, qui transmet au cerveau de fausses informations visant à empêcher le cerveau de commander la contraction de ces mêmes muscles.

Le dispositif conforme à l'invention peut comprendre les caractéristiques suivantes, prises séparément ou en combinaison :
- ledit bandeau comporte un détecteur de fréquence cardiaque, positionné sensiblement dans ledit plan de symétrie (ou positionné dans le plan de symétrie) de ladite structure (suivant un mode de réalisation particulier mais non limitatif), apte à se positionner soit sur un front de la personne soit sur une artère temporale, ledit détecteur de fréquence cardiaque étant apte à communiquer avec le module d'analyse et de commande. Ainsi, le dispositif conforme à l'invention surveille un paramètre supplémentaire ayant un lien avec le phénomène de bruxisme, par exemple pour mettre en oeuvre une action permettant de réduire le phénomène,
- ledit bandeau comprenant deux circuits temporaux, les deux circuits étant aptes à être positionnés sur les muscles temporaux d'une personne portant ladite structure et étant aptes à détecter des contractions musculaires temporales de ladite personne,
- ladite structure comporte une mémoire pour enregistrer lesdites données relatives aux contractions détectées et reçues par ledit module d'analyse et de commande,
- ladite structure comporte une batterie et en ce que ledit dispositif comporte une base, dissociée de ladite structure, ladite base étant apte à recharger ladite batterie,
- ladite base comporte un module de communication avec un serveur distant, et ladite base comporte un module de réception et de stockage de données, apte à recevoir et stocker les données enregistrées dans ladite mémoire de ladite structure, ledit module de communication étant apte à transmettre audit serveur lesdites données stockées. En dissociant les moyens de communication (avec un serveur distant) et la structure, la personne qui porte la structure n'est pas soumise à des ondes pouvant lui être néfastes, - lesdits circuits masséters comportent des capteurs de force flexibles piezorésistifs, lesdits capteurs de force étant aptes à détecter une contraction du muscle masséter. Par exemple, de tels capteurs sont commercialisé sous le nom de Flexiforce ^{®},
- lesdits circuits temporaux comportent des capteurs EMG aptes à détecter des contractions musculaires temporales de ladite personne et ou une activité neuronale au niveau des muscles temporaux de ladite personne,
- ledit détecteur de fréquence cardiaque comprend un oxymètre,
- ledit détecteur de fréquence cardiaque comprend des capteurs photopléthysmographiques.
- ladite structure comporte au moins un appareil permettant de capter (et éventuellement d'amplifier) les sons émis par ladite personne, ledit appareil étant apte à communiquer avec ledit module d'analyse et de commande. Un tel appareil est, par exemple commercialisé sous le nom de Sonetone^{®},
- la structure comporte un gyroscope ou un accéléromètre, apte à communiquer avec ledit module d'analyse et de commande.

L'invention concerne également un procédé de mise en oeuvre d'un dispositif tel que défini ci-avant, comportant les étapes suivantes :
- Détection de contractions musculaires temporales de ladite personne, et/ou
- Détection d'une contraction d'un muscle masséter sur lequel ladite extrémité libre de bras est positionnée,
- Analyse des contractions musculaires détectées par le module d'analyse et de commande, et
- Activation de l'un au moins desdits dispositifs de vibrations par le module d'analyse et de commande par commande dudit état de fonctionnement dudit au moins un desdits dispositifs de vibrations.

Suivant un mode de mise en oeuvre avantageux du procédé conforme à l'invention, Procédé selon la revendication 11, il peut être prévu que le procédé comporte une étape supplémentaire suivant laquelle :
- on détermine une fréquence cardiaque moyenne de ladite personne,
- on surveille une fréquence cardiaque réelle de ladite personne en déterminant, à intervalles de temps réguliers, des données de fréquence cardiaque à partir dudit détecteur de fréquence cardiaque,
- on compare les données de fréquence cardiaque déterminées à ladite fréquence cardiaque moyenne, et
- si les données de fréquence cardiaque déterminées sont supérieures à un pourcentage prédéterminé de ladite fréquence cardiaque moyenne, le module d'analyse et de commande active l'un au moins desdits dispositifs de vibrations par commande dudit état de fonctionnement dudit au moins un desdits dispositifs de vibrations.

Avantageusement, le procédé comporte une étape supplémentaire suivant laquelle on enregistre des données relatives aux contractions musculaires détectées ou non détectées pendant un temps donné.

En outre, suivant le procédé conforme à l'invention, lesdits données enregistrées peuvent être chargées dans la mémoire de ladite base indépendante de ladite structure et en ce que le module de communication de ladite base transmet, à un serveur distant, lesdites données enregistrées, ledit serveur distant traitant lesdites données pour les restituer, notamment via une application mise en oeuvre par un outil informatique, à ladite personne.

D'autres avantages et caractéristiques de l'invention apparaîtront à l'examen de la description détaillée d'un mode de mise en oeuvre nullement limitatif, et des dessins annexés, sur lesquels :
[Fig. 1] est une vue en perspective d'un dispositif conforme à l'invention, dont la structure est portée par une personne représentée en transparence,
[Fig. 2] est une vue en perspective de la structure du dispositif conforme à l'invention, montrée en figure 1, suivant un autre profil,
[Fig. 3] est encore une autre vue en perspective du dispositif montré en figure 1, dont la structure est portée par une personne représentée en transparence, montrant son profil droit,
[Fig. 4] est une vue en perspective d'une base que peut comporter un dispositif conforme à l'invention, associée à la structure montrée sur les figures 1 à 3,
[Fig. 5] est une vue éclatée de la structure montrée en figures 1 à 3,
[Fig. 6] est une représentation schématique d'un système assurant la mise en oeuvre d'un procédé conforme à l'invention, suivant lequel la personne est informée de l'évolution de son bruxisme durant la période d'utilisation du dispositif conforme à l'invention,
[Fig. 7] est un graphique illustrant une activité des muscles masséters gauche et droit, détectée par le dispositif conforme à l'invention, et plusieurs activations des moteurs générant des vibrations, notamment en fonction de l'activité des muscles masséters détectée, et
[Fig. 8] est un autre graphique illustrant une activité des muscles masséters gauche et droit, détectée par le dispositif conforme à l'invention, une activité cardiaque détectée par un oxymètre et plusieurs activations des moteurs générant des vibrations, notamment en fonction de l'activité des muscles masséters détectée.

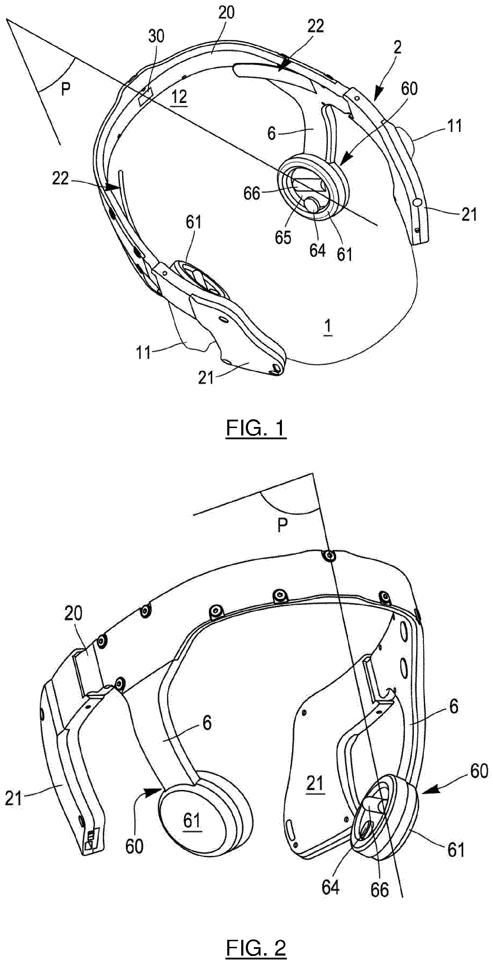

Les figures 1 à 3 montrent une partie d'un dispositif conforme à l'invention, qui est conçue pour identifier un ou plusieurs phénomènes de bruxisme chez une personne 1, pour collecter les données propres à ce phénomène et pour générer une action visant à réduire le phénomène.

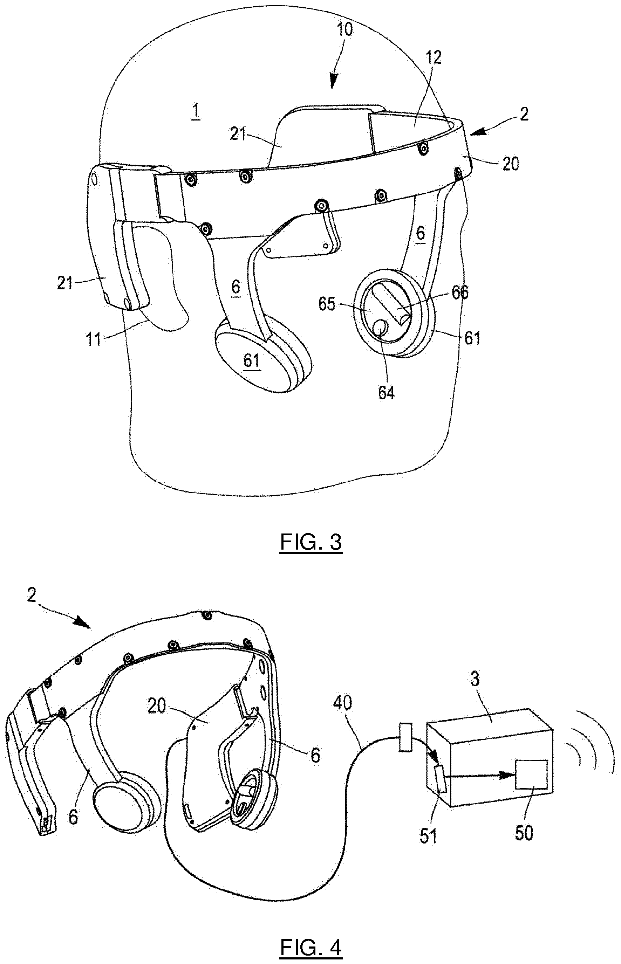

Cette partie se présente sous la forme d'une structure 2 en forme de casque et est associée avec une base 3, montrée en figure 4.

La base 3 permet de charger les données collectées par la structure en forme de casque pour les transmettre à un serveur distant 4, mettant en oeuvre une application (par exemple une application mobile), ce qui permet à la personne 1 de suivre, par exemple sur son smartphone 5 (mettant en oeuvre l'application), l'évolution des épisodes de bruxisme qu'elle subit et les résultats obtenus sur ces phénomènes en utilisant la structure 2 en forme de casque.

Il a été prévu de ne pas équiper la structure de moyens techniques permettant de transmettre à distance des données, pour éviter que la personne portant la structure ne soit soumise à des ondes pouvant lui être nocives.

La structure 2 montrée en figures 1 et 2, par exemple, comporte un bandeau 20 qui est conçue pour que, quand la personne porte la structure, le bandeau 20 soit placé sur le front d'une personne et s'étende de part et d'autre de la tête 10, au-dessus des oreilles 11 de la personne.

Le bandeau 20 présente deux parties symétriques par rapport à un plan de symétrie P, vertical quand la structure est portée par la personne 1.

Les extrémités du bandeau 20 sont solidaires de deux boitiers 21, qui se placent derrière les oreilles 11 de la personne.

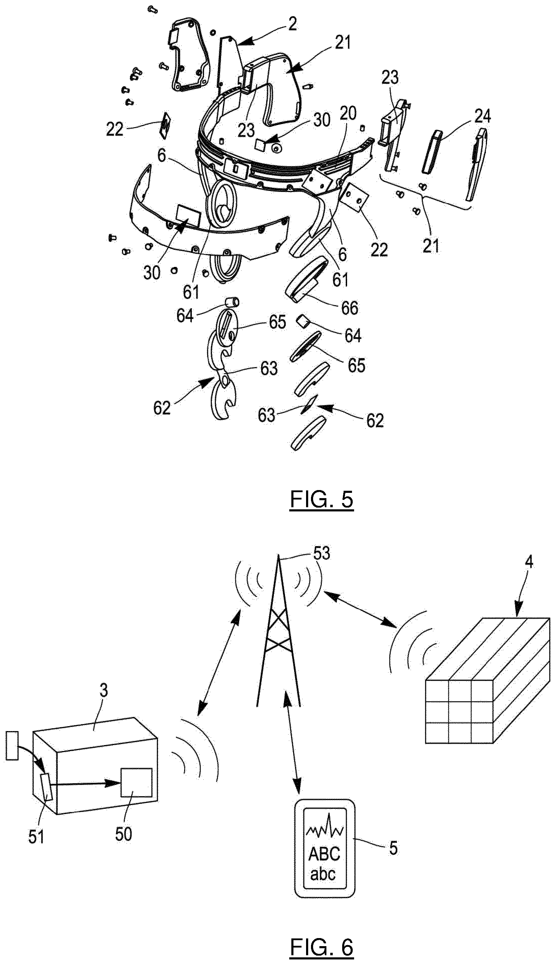

Les deux boitiers 21 permettent d'accueillir d'une part, deux appareils 23 de type SONETONE ^{®}, permettent de capter et éventuellement d'amplifier les sons émis par la personne 1 (chacun des boitiers comporte un appareil 23 - voir figure 5). L'utilité de ces appareils et leurs interactions avec d'autres éléments du dispositif conforme à l'invention sera expliquée par la suite.

D'autre part, l'un des boitiers 21 accueille une batterie 23, assurant l'apport en énergie de la structure pour son fonctionnement (figure 5).

Le bandeau 20 n'est pas conçu pour faire le tour de la tête, l'arrière de la tête 10 de la personne est ainsi libre et peut poser sur un support confortable sans pression sur l'arrière du crâne.

En outre, le bandeau 20 intègre deux circuits temporaux 22, que l'on voit en particulier sur la figure 5 : les deux circuits temporaux 22 sont placés dans le bandeau à une position suivant laquelle ils se placent contre les muscles temporaux de la personne 1quand elle porte la structure 2.

Les circuits temporaux 22 sont des capteurs de type EMG ou des capteurs Flexiforce ^{®} permettant d'enregistrer l'activité électrique d'un muscle ou d'un nerf, pour permettre la réalisation d'une électromyographie (encore appelé électroneuromyogramme) : ainsi lors d'un phénomène de bruxisme, lorsque les muscles temporaux se contractent, les circuits temporaux sont aptes à le détecter et à générer des données propres à l'activité des muscles temporaux.

Ces données sont enregistrées dans une mémoire que comporte également la structure : cette mémoire est incorporée dans une carte mère 25 placée dans un des boitiers 21.

Conformément à l'invention, la structure 1 comporte également deux bras 6, solidaires du bandeau 20, symétriques par rapport au plan P et qui s'étendent vers le bas depuis le bandeau pour présenter des extrémités libres 60 portant deux cassettes 61.

Chaque cassette 61 se trouve à une distance telle du bandeau qu'elle se place contre un muscle masséter du visage de la personne 1 quand la personne porte la structure 2.

Le contenu de chacune des cassettes 61 est visible ne particulier en figure 5 : chacune des cassettes 61 comporte un circuit 62, appelé « circuit masséter », apte à détecter une contraction du muscle masséter sur lequel ladite extrémité libre 60 de chaque bras 6 est positionnée.

Plus particulièrement, le circuit masséter 61 comporte des capteurs 63 de force flexibles piezorésistifs, connus par exemple dans le commerce sous le nom de capteurs Flexiforce ^{®}, qui sont aptes à détecter une contraction du muscle masséter et à émettre une donnée lors de cette détection.

Le capteur de contraction musculaire 63 comprend un circuit imprimé 64 sur une bande flexible, sur lequel prend appui un plot 65 : en se contractant, le muscle masséter exerce une pression sur le plot 64 qui appui sur le circuit 64, qui génère alors une donnée caractéristique de la contraction musculaire (force et durée par exemple).

Pour maintenir en position les plots 64 et les circuits 63, chaque cassette comporte une mousse 65 avec des découpes appropriées, accueillant ces éléments.

L'une des découpes des mousse 65 est conçue pour accueillir un dispositif de vibrations 60, apte à émettre des vibrations et pouvant fonctionner suivant un premier mode où il ne génère pas de vibrations (état de veille) et suivant un second mode où il génère des vibrations (étant de fonctionnement).

La commande du fonctionnement des dispositifs de vibration 66 est assurée par un module d'analyse et de commande, qui se trouve incorporé à la carte mère 25.

La carte mère 25, et son module d'analyse et de commande communique avec :
- lesdits circuits masséter 62,
- lesdits circuits temporaux 22 et
- lesdits dispositifs de vibration 66.

Plus particulièrement, ledit module d'analyse et de commande est conçu pour recevoir :
- les données en provenance des circuits masséter, sur la force et la durée des contractions musculaires détectées, et
- les données en provenance des circuits temporaux, relatives à l'activité électrique détectée dans les muscles temporaux.

Le module d'analyse et de commande est capable de collecter les données relatives à ces contractions ou à cette activité électrique, à les analyser pour y apporter une réponse éventuelle. Ces données et la réponse apportée par le module d'analyse et de commande sont enregistrées dans la mémoire de la structure 2, que comporte également la carte mère 25.

La réponse éventuellement apportée, susmentionnée, par le module d'analyse et de commande consiste à faire passer les dispositifs de vibration de leur état de veille à leur état de fonctionnement, ou inversement, en fonction de données seuils prédéterminée et renseignées dans la carte mère : il s'agit de déterminer si, en fonction de l'intensité des contractions musculaire et de leur durée de contraction, il est nécessaire de générer des vibrations au niveau des muscles masséter ou non.

Les vibrations générées présentent des fréquences de 10 à 150 Hz et des amplitudes de 0,1 à 5mm : ces fourchettes de fréquences et d'amplitude sont telles qu'elles sont détectables par les muscles masséters et par les neurones qui en assurent la contraction, pour permettre de transmettre au cerveau des données visant à le tromper et à réduire l'activité du cerveau à l'origine de la contraction des muscles masséters et temporaux qui caractérisent les épisodes de bruxisme.

Ainsi, après avoir identifié des contractions suffisamment fortes et longues des muscles masséter et/ou une activité électrique dans les muscles temporaux, le module d'analyse et de commande ordonne le fonctionnement des dispositifs de vibration qui indiquent au cerveau que les muscles masséter ou temporaux sont contractés, de sorte que le cerveau arrête de commander cette contraction : en effet, en recevant des messages générées par les vibrations captées par les muscles, le cerveau est trompé et cesse de générer des ordres à l'origine du phénomène de bruxisme.

Il est à noter que, conformément au mode de réalisation présenté et suivant une particularité de cet exemple, le module d'analyse et de commande communique également avec un autre dispositif que comporte la structure, dans le bandeau 20 : le bandeau comporte un détecteur de fréquence cardiaque 30, positionné sensiblement dans ledit plan de symétrie P (ou positionné dans ledit plan de symétrie) de ladite structure 2, apte à se positionner sur un front 12 de la personne.

Il devra être compris que la position du détecteur de fréquence cardiaque est ici donnée à titre d'exemple de mise en oeuvre. Suivant d'autres modes de réalisation d'un dispositif conforme à l'invention, le détecteur de fréquence cardiaque pourrait être positionné sur une artère temporale, par exemple (donc non positionné dans le plan de symétrie), sans sortir du cadre de l'invention.

Le détecteur de fréquence cardiaque 30 communique avec le module d'analyse et de commande et consiste en un oxymètre, c'est-à-dire un appareil qui mesure la fréquence cardiaque et la concentration d'oxygène dans le sang.

Cet oxymètre présente ainsi l'avantage de surveiller un autre paramètre pouvant indiquer un phénomène de bruxisme, puisqu'il a été démontré un lien entre le phénomène de bruxisme et une modification du rythme cardiaque des personnes vivant ces phénomènes : c'est ainsi que le rythme cardiaque d'une personne peut augmenter de 40 %.

L'oxymètre 30, qui communique avec le module d'analyse et de commande, peut renseigner le module avec une information relative à une augmentation du rythme cardiaque anormal. En réponse à cette information, le module d'analyse et de commande peut également commander l'état de fonctionnement des dispositifs de vibration, pour tromper le cerveau et ainsi contribuer à un retour à la normale du rythme cardiaque de la personne.

Les appareils 23 de détection de bruits sont également connectés avec le module d'analyse et de commande. Ils sont également connectés avec la mémoire de la structure 2 pour enregistrer les divers bruits faits par la personne, par exemple un ronflement, un grincement de dents, le bruit de la respiration de la personne pouvant s'arrêter et révéler des phénomènes d'apnées du sommeil.

Un ultime dispositif de type gyroscope, non visible sur les figures, équipe également la structure 2 du dispositif conforme à l'invention : il est inclus dans le bandeau 2, par exemple, et permet de détecter les mouvements de tête de la personne pendant son sommeil, d'enregistrer ces informations de mouvements sous forme de données dans la mémoire de la carte mère 25.

De façon générale, toutes les informations collectées par les dispositifs qui équipent la structure 2 sont enregistrées dans le module d'analyse et de commande de la structure, pour permettre de délivrer à la personne non seulement les informations relatives au phénomène de bruxisme qu'elle subit, son évolution suite à l'utilisation du dispositif conforme à l'invention, mais également d'autres informations sur des phénomènes qui peuvent être en lien avec le bruxisme ou non.

Pour permettre de transférer ces informations, le dispositif comporte une base 3, annexe à la structure, qui a été présentée plus en avant dans ce récit, qui est conçue non seulement pour récupérer les données enregistrées dans la mémoire de la structure 2, mais également pour transmettre ces données afin qu'elles soient restituées à la personne, par exemple via une application smartphone ou via un site Internet.

La structure n'est pas conçue, en effet, pour transmettre directement les informations à un serveur distant : pour ce faire, il faudrait équiper la structure d'un module de communication qui émet des ondes. Même si ce mode de réalisation n'est pas exclu des modes de réalisation conformes à l'invention, il a été choisir de réaliser une base distincte, équipé d'un tel module de communication, qui est placé à distance de la personne quand elle porte la structure, pour ne pas mettre en contact la personne et des ondes de transmission éventuelle entre la base et un serveur distant.

La base permet donc de récupérer les données enregistrées dans la structure et comporte, pour ce faire un module de communication, ainsi qu'un module de réception et de stockage des données en provenance de la structure 2 : ces modules portant respectivement les références 50 et 51 ont été schématiquement représentés par des rectangles en traits pointillés sur la figure 4.

Le module de communication 50 assure le transfert des données, par exemple via un réseau de communication longue distance 53 à un serveur distant 4 qui met en oeuvre une application smartphone ou Internet dédiée à l'invention.

On supposera, dans l'exemple de mise en oeuvre présentement décrit, que l'application est opérationnelle sur le smartphone 5 de la personne.

Les informations de mise à jour de l'application et des données qu'elle comprend, concernant la personne utilisant le dispositif conforme à l'invention, sont transmises au smartphone 5 également par le réseau de communication longue distance 53.

La base 3 permet également de recharger la batterie 24 de la structure 2. Pour recharger la batterie, la base 3 est équipée d'une prise USB, qui se raccorde à un câble USB (référence 40, figure 4) branché sur le boîtier 21 de la structure 2 comportant la batterie 24.

Il devra être compris que le chargement de la batterie pourrait se recharger par d'autres moyens que par un branchement, sans sortir du cadre de l'invention.

Il va maintenant être fait référence à un procédé d'utilisation du dispositif décrit ci-avant.

Une personne atteinte de bruxisme souhaite réaliser une première séance avec le dispositif conforme à l'invention.

La structure 2 est positionnée sur la tête 1 de la personne, et la structure est mise en état de fonctionnement en appuyant sur le bouton de contact, par exemple.

La personne 1 s'allonge et se laisse gagner par le sommeil.

Quand un phénomène de bruxisme commence, la personne commence par serrer ses deux mâchoires l'une sur l'autre, contractant ainsi les muscles masséter.

Les muscles temporaux génèrent également une activité électrique.

Les circuits masséters et temporaux transmettent alors des données relatives à la contraction des muscles temporaux et/ou masséter au module d'analyse et de commande de la structure.

Parallèlement, il se peut que la personne émette des sons produits par le grincement des dents qui frottent les unes contre les autres : dans ce cas, ces évènements sont captés par les appareils de type SONOTONE ^{®} et ils sont également enregistrés par ce module d'analyse et de commande.

Ces sons peuvent être associés à des données enregistrées à la place du son lui-même, par exemple l'intensité du son et sa durée peut être enregistrées.

La fréquence cardiaque peut également être enregistrée en temps réelle par le module d'analyse et de commande, grâce à l'oxymètre positionné sur le front 12 de la personne.

Si les données relatives aux mouvements des muscles masséters et temporaux sont caractéristiques d'un phénomène de bruxisme, ou si la fréquence cardiaque de la personne, excède une fréquence cardiaque seuil prédéterminée, alors le module d'analyse et de commande met en fonctionnement les dispositifs de vibration, qui met en contact les muscles masséter avec les vibrations.

On considère une donnée comme étant caractéristique d'un phénomène de bruxisme si l'intensité et la durée des contractions musculaires sont conformes celles identifiées comme témoignant d'un phénomène de bruxisme dans une base de données que comporte la carte mère 25 de la structure
Pour identifier les données comme étant caractéristiques d'un phénomène de bruxisme, les donnes collectées par le module d'analyse et de commande sont comparées aux données de la base de données

Il devra être compris que, suivant une variante de réalisation, il est possible de faire fonctionner le dispositif sans avoir à comparer les données collectées avec des données de la base de données : le dispositif peut fonctionner en générant des vibrations dès qu'une activité est détectée au niveau des masséters et/ou des temporaux. Les vibrations, générées par les dispositifs de vibrations dans les circuits masséter, sont perçues par les muscles masséters, qui se détendent sous leur action. De plus, le réseau neuronal des muscles masséter captent ces vibrations et envoie au cerveau un signal suivant lequel les muscles sont stimulés, de sorte que le cerveau, trompé, arrête de commander la contraction de ces muscles masséters.

La commande du fonctionnement des dispositifs vibratoires peut être activée ou désactivée en surveillant également le rythme cardiaque de la personne.

L'oxymètre permet alors de détecter que le rythme cardiaque de la personne baisse jusqu'à un seuil prédéterminé en dessous duquel le module d'analyse et de commande arrête le fonctionnement des dispositifs de vibration.

Le seuil au-dessous duquel les dispositifs vibratoires sont désactivés et au-dessus duquel les dispositifs vibratoires sont activés est déterminé pour chaque personne.

Par exemple, lors d'une séance, en début de séance, le module d'analyse et de commande relève la fréquence cardiaque de la personne à intervalles de temps régulier et en fait une moyenne pour déterminer une fréquence cardiaque moyenne.

Le module de commande va déterminer la valeur seuil en multipliant cette fréquence cardiaque moyenne par 1,3, pour que les dispositifs vibratoires soient déclenchés si la fréquence cardiaque de la personne est identifiée comme étant supérieure à 20% de sa valeur moyenne, par exemple.

Les données reçues par le module d'analyse et de commande sont enregistrées pendant un temps prédéterminé.

Ce temps peut être de huit heures, par exemple. Plusieurs séances peuvent également être enregistrées par la structure avant que les données ne soient collectées par la base 3, lors de la recharge de la batterie de la structure.

La base 3 transmet au serveur distant les données qu'elle a collectées, par exemple en les associant avec un identifiant utilisateur : ainsi, le serveur renseigne uniquement le compte utilisateur avec les données qu'il reçoit. Ces données sont alors restituées, par exemple sous forme de courbes ou de tableau à l'utilisateur (la personne 1) qui peut ainsi suivre, sur son smartphone, l'évolution des épisodes de bruxisme qu'elle peut faire, leur durée leur intensité, au fur et à mesure de l'utilisation du dispositif conforme à l'invention.

Il va maintenant être fait référence aux résultats de deux séances réalisées avec le dispositif conforme à l'invention, et qui peuvent être enregistrées et transmises à l'utilisateur du dispositif, notamment grâce à l'utilisation de l'application mise en oeuvre sur son smartphone.

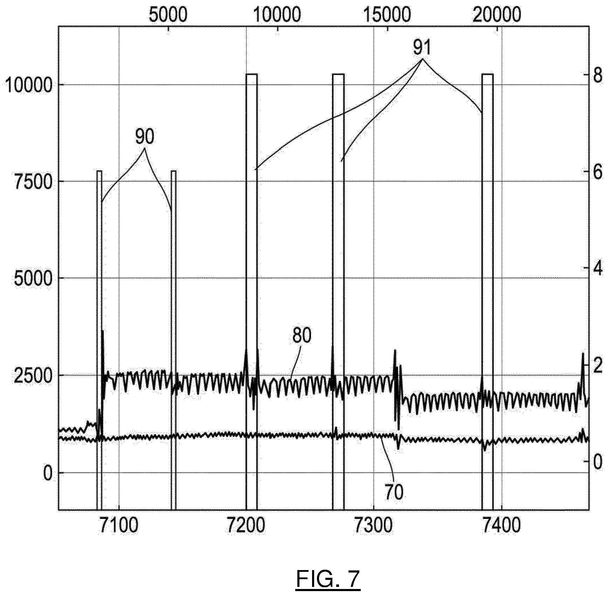

La figure 7 est un graphique illustrant des évènements survenus lors d'une séance avec le dispositif conforme à l'invention.

Le temps passé, en secondes, est indiqué en abscisse, les ordonnées sur la gauche correspondent au nombre de bits enregistrés, générés par une activité électrique ou musculaires des masséters et les ordonnées sur la droite correspondent à l'intensité des vibrations générées par le dispositif de vibrations, suivant le mode de vibrations qui est activé. La référence 80 correspond à l'activité du masséter droit enregistrée, la référence 70 correspond à l'activité du masséter gauche enregistrée.

On remarque une première série d'activations des dispositifs de vibrations 66, correspondant aux courbes portant la référence 90 : les dispositifs de vibrations 66 sont activés deux fois.

Il s'agit d'un premier mode de fonctionnement suivant lequel les dispositifs de vibration sont activés, dès lors que les capteurs 63 détectent une activité musculaire masséter

La référence 91 correspond à un second mode de fonctionnement : les dispositifs de vibration 66 sont déclenchés trois fois pendant dix secondes, ces trois déclenchements étant générés suite à la détection, par un des capteurs 63 d'une activité des masséters gauche ou droit pendant au moins 2 secondes.

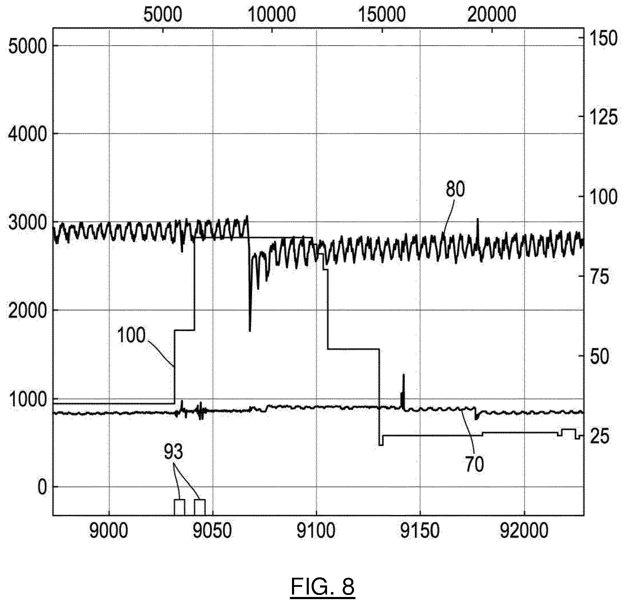

La figure 8 est un autre graphique, illustrant les évènements lors d'une autre séance mettant en oeuvre le dispositif conforme à l'invention.

Le temps passé, en secondes, est indiqué en abscisse, les ordonnées sur la gauche correspondent au nombre de bits enregistrés, générés par une activité électrique ou musculaires des masséters et les ordonnées sur la droite correspondent à l'intensité des vibrations générées par le dispositif de vibration.

On remarque que l'activité des masséters gauche et droit n'est pas significative (les courbes 70 et 80 sont régulières et sensiblement plates, ou plates). Néanmoins, on remarque l'activation des dispositifs de vibration à deux reprises (référence 93) lorsque le détecteur de fréquence cardiaque, illustré par la courbe portant la référence 100, enregistre une montée fulgurante du rythme cardiaque (augmentation du rythme cardiaque de plus de 20%).

D'autres graphiques révélant d'autres résultats pourraient être obtenus : par exemple, ils pourraient illustrer l'activité des muscles temporaux et la réponse éventuelle des dispositifs de vibrations 60.

Il peut être accessible par l'utilisateur sur son smartphone 5.

L'activité des muscles temporaux n'a pas été présentée sur les figures 7 et 8 afin d'en faciliter la lecture.

On comprend de ce qui précède comment l'invention permet non seulement de détecter les phénomènes de bruxisme mais également de les traiter et de suivre leur évolution au cours du temps.

Il devra être compris que l'invention n'est pas limitée au mode de réalisation spécifiquement décrit et représenté sur les figures, et qu'elle s'étend à la mise en oeuvre de tout moyen équivalent.

## Revendications

1. Dispositif pour aider à lutter contre le bruxisme, comportant une structure (2) conçue pour être portée sur la tête (1) d'une personne, ladite structure (2) comportant un bandeau (20) présentant un plan de symétrie (P) de part et d'autre duquel il s'étend,
ladite structure (2) comportant deux bras (6) symétriques par rapport audit plan de symétrie (P), les deux dits bras (6) étant reliés audit bandeau (20) par une première extrémité de bras, ledit dispositif étant **caractérisé en ce que** les deux dits bras (6) s'étendent vers le bas depuis le bandeau et présentant chacun une second extrémité libre (60) de bras, ladite second extrémité libre (60) de bras de chacun des bras étant à une distance telle du bandeau qu'elle est apte à se positionner contre un muscle masséter quand ladite personne porte ladite structure (2) et ladite seconde extrémité (60) comportant un circuit masséter (63), apte à détecter une contraction du muscle masséter sur lequel ladite extrémité libre (60) de bras est positionnée, ladite extrémité libre comportant un dispositif de vibrations (66) apte à adopter soit un état de fonctionnement suivant lequel il émet des vibrations, soit un état de veille suivant lequel il n'émet pas de vibration,
ledit dispositif comportant un module (25) d'analyse et de commande, communiquant avec lesdits circuits masséter (63), et avec lesdits dispositifs de vibration (66), ledit module (25) d'analyse et de commande étant apte à collecter et analyser des données relatives audites contractions détectées, et apte à commander le passage des dispositifs de vibrations (66) dudit état de veille audit état de fonctionnement, et inversement.

2. Dispositif selon la revendication 1, **caractérisé en ce que** ledit bandeau (20) comporte un détecteur (30) de fréquence cardiaque apte à se positionner soit sur un front (12) de la personne soit sur une artère temporale, ledit détecteur (30) de fréquence cardiaque étant apte à communiquer avec le module (25) d'analyse et de commande.

3. Dispositif selon l'une quelconque des revendications précédentes, **caractérisé en ce que** ledit bandeau (20) comprend deux circuits temporaux (22), les deux circuits temporaux (22) étant aptes à être positionnés sur les muscles temporaux de ladite personne portant ladite structure (2) et étant aptes à détecter des contractions musculaires temporales de ladite personne.

4. Dispositif selon l'une quelconque des revendications précédentes, **caractérisé en ce que** ladite structure (2) comporte une mémoire pour enregistrer lesdites données relatives aux contractions détectées et reçues par ledit module (25) d'analyse et de commande.

5. Dispositif selon l'une quelconque des revendications précédentes, **caractérisé en ce que** ladite structure (2) comporte une batterie (24) et **en ce que** ledit dispositif comporte une base (3), dissociée de ladite structure (2), ladite base (3) étant apte à recharger ladite batterie (24).

6. Dispositif selon la revendication 4 et selon la revendication 5, **caractérisé en ce que** ladite base (3) comporte un module (50) de communication avec un serveur distant (4), et **en ce que** ladite base (3) comporte un module (51) de réception et de stockage de données, apte à recevoir et stocker les données enregistrées dans ladite mémoire de ladite structure (2), ledit module de communication (50) étant apte à transmettre audit serveur distant (4) lesdites données stockées.

7. Dispositif selon l'une quelconque des revendications précédentes, **caractérisé en ce que** lesdits circuits masséters (63) comportent des capteurs de force flexibles piezorésistifs (63, 64), lesdits capteurs de force (63, 64) étant aptes à détecter une contraction du muscle masséter.

8. Dispositif selon l'une quelconque des revendications précédentes, **caractérisé en ce que** lesdits circuits temporaux (22) comportent des capteurs EMG aptes à détecter des contractions musculaires temporales de ladite personne et ou une activité neuronale au niveau des muscles temporaux de ladite personne.

9. Dispositif selon la revendication 2, **caractérisé en ce que** ledit détecteur de fréquence cardiaque (30) comprend un oxymètre.

10. Dispositif selon la revendication 2, **caractérisé en ce que** ledit détecteur de fréquence cardiaque comprend des capteurs photopléthysmographiques.

11. Dispositif selon l'une quelconque des revendications précédentes, **caractérisé en ce que** ladite structure (2) comporte au moins un appareil (23) permettant de capter, et éventuellement d'amplifier, les sons émis par ladite personne, ledit au moins un appareil (23) étant apte à communiquer avec ledit module (25) d'analyse et de commande.

12. Dispositif selon l'une quelconque des revendications précédentes, **caractérisé en ce que** la structure (2) comporte un gyroscope ou un accéléromètre, apte à communiquer avec ledit module d'analyse et de commande.

13. Procédé de mise en oeuvre d'un dispositif selon l'une quelconque des revendications précédentes comportant au moins les caractéristiques de la revendication 3, ledit procédé comportant les étapes suivantes :
- Détection de contractions musculaires temporales de ladite personne par lesdits circuits temporaux (22), et/ou
- Détection d'une contraction d'un muscle masséter sur lequel ladite extrémité libre de bras est positionnée par lesdits circuits masséters (63),
- Analyse des contractions musculaires détectées par le module (25) d'analyse et de commande, et
- Activation de l'un au moins desdits dispositifs de vibrations (66) par le module (25) d'analyse et de commande, par commande dudit état de fonctionnement dudit au moins un desdits dispositifs de vibrations (66).

14. Procédé selon la revendication 13 , mettant en oeuvre un dispositif selon la revendication 2, 9 ou 10, **caractérisé en ce qu'**il comporte une étape supplémentaire suivant laquelle :
- on détermine une fréquence cardiaque moyenne de ladite personne,
- on surveille une fréquence cardiaque réelle de ladite personne en déterminant, à intervalles de temps réguliers, des données de fréquence cardiaque à partir dudit détecteur de fréquence cardiaque (30),
- on compare les données de fréquence cardiaque déterminées à ladite fréquence cardiaque moyenne, et
- si les données de fréquence cardiaque déterminées sont supérieures à un pourcentage prédéterminé de ladite fréquence cardiaque moyenne, le module (25) d'analyse et de commande active l'un au moins desdits dispositifs (66) de vibrations par commande dudit état de fonctionnement dudit au moins un desdits dispositifs de vibrations.

15. Procédé selon l'une quelconque des revendications 13 ou 14, **caractérisé en ce que** l'on enregistre des données relatives aux contractions musculaires détectées ou non détectées pendant un temps donné.

16. Procédé selon la revendication 15 , mettant en oeuvre le dispositif selon la revendication 5, **caractérisé en ce que** lesdits données enregistrées sont chargées dans le module (51) de réception et de stockage de données de la base (3),et **en ce que** le module de communication (50) de ladite base (30) transmet, à un serveur distant (4), lesdites données enregistrées, ledit serveur distant (4) traitant lesdites données enregistrées pour les restituer, notamment via une application mise en oeuvre par un outil informatique, à ladite personne.

## Patentansprüche

1. Vorrichtung zur Unterstützung der Bekämpfung von Bruxismus, umfassend eine Struktur (2), die geeignet ist, auf dem Kopf (1) einer Person getragen zu werden, wobei die Struktur (2) ein Band (20) umfasst, das eine Symmetrieebene (P) aufweist, von der aus es sich beidseitig erstreckt, wobei die Struktur (2) zwei Arme (6) umfasst, die symmetrisch bezüglich der Symmetrieebene (P) sind, wobei die zwei Arme (6) über ein erstes Armende mit dem Band (20) verbunden sind, wobei die Vorrichtung **dadurch gekennzeichnet ist, dass** sich die beiden Arme (6) vom Band aus nach unten erstrecken und jeweils ein zweites, freies Ende (60) aufweisen, wobei sich das zweite, freie Armende (60) jeweils in einem solchen Abstand von dem Band befindet, dass es sich gegen einen Kaumuskel positionieren kann, wenn die Person die Struktur (2) trägt, und das zweite Ende (60) eine Kauschaltung (63) trägt, die eine Kontraktion des Kaumuskels nachweisen kann, auf dem das freie Armende (60) positioniert ist, wobei das freie Ende eine Vibrationsvorrichtung (66) umfasst, die entweder einen Funktionszustand, gemäß dem sie Schwingungen aussendet, oder einen Wachzustand, gemäß dem sie keine Schwingungen aussendet, annehmen kann,
wobei die Vorrichtung ein Analyse- und Steuermodul (25) umfasst, das mit den Kauschaltungen (63) und mit den Vibrationsvorrichtungen (66) kommuniziert, wobei das Analyse- und Steuermodul (25) in der Lage ist, die Daten bezüglich der nachgewiesenen Kontraktionen zu sammeln und zu analysieren, und in der Lage ist, den Übergang der Vibrationsvorrichtungen (66) vom Funktionszustand zum Wachzustand und umgekehrt zu steuern.

2. Vorrichtung gemäß Anspruch 1, **dadurch gekennzeichnet, dass** das Band (20) einen Herzfrequenzdetektor (30) umfasst, der sich entweder auf der Stirn (12) der Person oder auf einer Schläfenarterie positionieren kann, wobei der Herzfrequenzdetektor (30) mit dem Analyse- und Steuermodul (25) kommunizieren kann.

3. Vorrichtung gemäß einem der vorstehenden Ansprüche, **dadurch gekennzeichnet, dass** das Band (20) zwei Schläfenschaltungen (22) umfasst, wobei die zwei Schläfenschaltungen (22) auf den Schläfenmuskeln der Person, die die Struktur (2) trägt, positioniert werden können und Kontraktionen der Schläfenmuskeln dieser Person nachweisen können.

4. Vorrichtung gemäß einem der vorstehenden Ansprüche, **dadurch gekennzeichnet, dass** die Struktur (2) einen Speicher umfasst, um die von dem Analyse- und Steuermodul (25) empfangenen Daten in Bezug auf die nachgewiesenen Kontraktionen aufzuzeichnen.

5. Vorrichtung gemäß einem der vorstehenden Ansprüche, **dadurch gekennzeichnet, dass** die Struktur (2) eine Batterie (24) umfasst, und dadurch, dass die Vorrichtung eine Basis (3) umfasst, die von der Struktur (2) losgelöst ist, wobei die Basis (3) die Batterie (24) wiederaufladen kann.

6. Vorrichtung gemäß Anspruch 4 und 5, **dadurch gekennzeichnet, dass** die Basis (3) ein Modul (50) zur Kommunikation mit einem Fernserver (4) umfasst, und dadurch, dass die Basis (3) ein Modul (51) zum Empfangen und Speichern von Daten umfasst, das die in dem Speicher der Struktur (2) aufgezeichneten Daten aufnehmen und speichern kann, wobei das Kommunikationsmodul (50) die gespeicherten Daten an den Fernserver (4) senden kann.

7. Vorrichtung gemäß einem der vorstehenden Ansprüche, **dadurch gekennzeichnet, dass** die Kauschaltungen (63) flexible piezoresistive Kraftsensoren (63, 64) umfassen, wobei die Kraftsensoren (63, 64) eine Kontraktion des Kaumuskels nachweisen können.

8. Vorrichtung gemäß einem der vorstehenden Ansprüche, **dadurch gekennzeichnet, dass** die Schläfenschaltungen (22) EMG-Sensoren umfassen, die Kontraktionen des Schläfenmuskels der Person und/oder eine neuronale Aktivität an den Schläfenmuskeln der Person nachweisen können.

9. Vorrichtung gemäß Anspruch 2, **dadurch gekennzeichnet, dass** der Herzfrequenzdetektor (30) ein Oximeter umfasst.

10. Vorrichtung gemäß Anspruch 2, **dadurch gekennzeichnet, dass** der Herzfrequenzdetektor photoplethysmographische Sensoren umfasst.

11. Vorrichtung gemäß einem der vorstehenden Ansprüche, **dadurch gekennzeichnet, dass** die Struktur (2) wenigstens eine Apparatur (23) umfasst, mit der die von der Person ausgehenden Geräusche nachgewiesen und gegebenenfalls verstärkt werden können, wobei die wenigstens eine Apparatur (23) mit dem Analyse- und Steuermodul (25) kommunizieren kann.

12. Vorrichtung gemäß einem der vorstehenden Ansprüche, **dadurch gekennzeichnet, dass** die Struktur (2) ein Gyroskop oder einen Beschleunigungsmesser umfasst, das bzw. der mit dem Analyse- und Steuermodul kommunizieren kann.

13. Verfahren zur Anwendung einer Vorrichtung gemäß einem der vorstehenden Ansprüche, die wenigstens die Merkmale von Anspruch 3 umfasst, wobei das Verfahren die folgenden Schritte umfasst:
- Nachweis von Kontraktionen des Schläfenmuskels der Person durch die Schläfenschaltungen (22) und/oder
- Nachweis einer Kontraktion eines Kaumuskels, auf dem das freie Armende positioniert ist, durch die Kauschaltungen (63),
- Analyse der von dem Analyse- und Steuermodul (25) nachgewiesenen Muskelkontraktionen und
- Aktivierung wenigstens einer der Vibrationsvorrichtungen (66) durch das Analyse- und Steuermodul (25) auf Befehl des Funktionszustands des wenigstens einen der Vibrationsvorrichtungen (66).

14. Verfahren gemäß Anspruch 13, das eine Vorrichtung gemäß Anspruch 2, 9 oder 10 anwendet, **dadurch gekennzeichnet, dass** es einen zusätzlichen Schritt umfasst, gemäß dem:
- man eine mittlere Herzfrequenz der Person bestimmt,
- man eine reale Herzfrequenz der Person überwacht, indem man in regelmäßigen Zeitabständen Herzfrequenzdaten von dem Herzfrequenzdetektor (30) bestimmt,
- man die bestimmten Herzfrequenzdaten mit der mittleren Herzfrequenz vergleicht und
- wenn die bestimmten Herzfrequenzdaten größer als ein vorbestimmter Prozentsatz der mittleren Herzfrequenz sind, aktiviert das Analyse- und Steuermodul (25) wenigstens eine der Vibrationsvorrichtungen (66) auf Befehl des Funktionszustands des wenigstens einen der Vibrationsvorrichtungen.

15. Verfahren gemäß einem der Ansprüche 13 oder 14, **dadurch gekennzeichnet, dass** man Daten in Bezug auf nachgewiesene oder nicht nachgewiesene Muskelkontraktionen über einen gegebenen Zeitraum aufzeichnet.

16. Verfahren gemäß Anspruch 15, das die Vorrichtung gemäß Anspruch 5 anwendet, **dadurch gekennzeichnet, dass** die aufgezeichneten Daten in das Modul (51) zum Empfangen und Speichern von Daten der Basis (3) geladen werden, und dadurch, dass das Kommunikationsmodul (50) der Basis (3) die aufgezeichneten Daten an einen Fernserver (4) sendet, wobei der Fernserver (4) die aufgezeichneten Daten verarbeitet, um sie insbesondere über eine App, die von einem IT-Werkzeug ausgeführt wird, an die Person zurückzugeben.

## Claims

1. A device to help combat bruxism, including a structure (2) designed to be worn on the head (1) of a person, said structure (2) including a headband (20) having a plane of symmetry (P) on either side of which it extends,
said structure (2) including two arms (6) symmetrical with respect to said plane of symmetry (P), said two arms (6) being connected to said headband (20) by a first arm end, said device being **characterised in that** the two said arms (6) extend downwards from the headband and each have a second free arm end (60), said second free arm end (60) of each of the arms being at such a distance from the headband that it is able to be positioned against a masseter muscle when said person is wearing said structure (2) and said second end (60) including a masseter circuit (63), able to detect contraction of the masseter muscle on which said free arm end (60) is positioned, said free end including a vibration device (66) able to adopt either an operating state wherein it emits vibrations, or a standby state wherein it does not emit vibrations,
said device including an analysis and control module (25), communicating with said masseter circuits (63), and with said vibration devices (66), said analysis and control module (25) being able to collect and analyse data relating to said contractions detected, and able to control shifting of the vibration devices (66) from said standby state to said operating state, and vice versa.

2. The device according to claim 1, **characterised in that** said headband (20) includes a heart rate detector (30) able to be positioned either on the person's forehead (12) or on a temporal artery, said heart rate detector (30) being able to communicate with the analysis and control module (25) .

3. The device according to any of the preceding claims, **characterised in that** said headband (20) comprises two temporal circuits (22), the two temporal circuits (22) being able to be positioned on the temporal muscles of said person wearing said structure (2) and being able to detect temporal muscle contractions of said person.

4. The device according to any of the preceding claims, **characterised in that** said structure (2) includes a memory for storing said data relating to the contractions detected and received by said analysis and control module (25) .

5. The device according to any of the preceding claims, **characterised in that** said structure (2) includes a battery (24) and **in that** said device includes a base (3), dissociated from said structure (2), said base (3) being able to recharge said battery (24).

6. The device according to claim 4 and according to claim 5, **characterised in that** said base (3) includes a module (50) for communication with a remote server (4), and **in that** said base (3) includes a module (51) for receiving and storing data, able to receive and store the data recorded in said memory of said structure (2), said communication module (50) being able to transmit said data stored to said remote server (4).

7. The device according to any of the preceding claims, **characterised in that** said masseter circuits (63) include flexible piezoresistive force sensors (63, 64), said force sensors (63, 64) being able to detect contraction of the masseter muscle.

8. The device according to any of the preceding claims, **characterised in that** said temporal circuits (22) include EMG sensors able to detect temporal muscle contractions of said person and/or neuronal activity at the temporal muscles of said person.

9. The device according to claim 2, **characterised in that** said heart rate detector (30) comprises an oximeter.

10. The device according to claim 2, **characterised in that** said heart rate detector comprises photoplethysmographic sensors.

11. The device according to any of the preceding claims, **characterised in that** said structure (2) includes at least one apparatus (23) for sensing, and possibly amplifying, sounds emitted by said person, said at least one apparatus (23) being able to communicate with said analysis and control module (25).

12. The device according to any of the preceding claims, **characterised in that** the structure (2) includes a gyroscope or accelerometer, able to communicate with said analysis and control module.

13. A method for implementing a device according to any of the preceding claims including at least the characteristics of claim 3, said method including the following steps of:
- detecting temporal muscle contractions of said person by said temporal circuits (22), and/or
- detecting contraction of a masseter muscle on which said free arm end is positioned by said masseter circuits (63),
- analysing the muscle contractions detected by the analysis and control module (25), and
- activating at least one of said vibration devices (66) by the analysis and control module (25), by controlling said operating state of said at least one of said vibration devices (66).

14. The method according to claim 13, implementing a device according to claim 2, 9 or 10, **characterised in that** it includes an additional step wherein:
- an average heart rate of said person is determined,
- an actual heart rate of said person is monitored by determining, at regular time intervals, heart rate data from said heart rate detector (30),
- the heart rate data determined are compared with said mean heart rate, and
- if the heart rate data determined are greater than a predetermined percentage of said mean heart rate, the analysis and control module (25) activates at least one of said vibration devices (66) by controlling said operating state of said at least one of said vibration devices.

15. The method according to any of claims 13 or 14, **characterised in that** data relating to muscle contractions detected or not detected for a given time are recorded.

16. The method according to claim 15, implementing the device according to claim 5, **characterised in that** said data recorded are loaded into the data reception and storage module (51) of the base (3), and **in that** the communication module (50) of said base (3) transmits said data recorded to a remote server (4), said remote server (4) processing said data recorded in order to reproduce them, especially via an application implemented by a computational tool, to said person.
